# EUROPEAN PATENT SPECIFICATION

(11) **EP 3 092 214 B1**
(45) Date of publication and mention of the grant of the patent: **20.06.2018**
(21) Application number: 15714015.3
(22) Date of filing: 08.01.2015
(51) Int. Cl.: C07C 291/04, C11D 1/75, C11D 3/36

(54) **NITROSAMINE/NITRITE INHIBITION IN TERTIARY ALKYLAMINE OXIDE COMPOSITIONS**
NITROSAMIN/NITRITHEMMUNG IN TERTIÄREN ALKYLAMINOXIDZUSAMMENSETZUNGEN
INHIBITION DES NITROSAMINES/NITRITES DANS DES COMPOSITIONS D'OXYDE D'ALKYLAMINE TERTIAIRE

(30) Priority: 08.01.2014 BE 201400010
(43) Date of publication of application: 16.11.2016
(73) Proprietor: EOC Surfactants N.V., 9940 Evergem (BE)
(72) Inventor: DE RYCKE, Daisy Sofie, 9690 Kluisbergen (BE); UYTTERSPROT, Katrijn, 9420 Erpe-Mere (BE); TAELMAN, Marie Claire Leona Hilda, 9790 Wortegem-Petegem (BE); VEHENT, Dirk Cyriel, 9160 Lokeren (BE)
(74) Representative: Brantsandpatents bvba
(86) International application number: PCT/IB2015/050149
(87) International publication number: WO 2015/104673

(56) References cited:
- EP-A1- 0 608 873
- US-A- 5 023 376
- US-A- 5 442 113
- K. MOEDRITZER, ET AL.: "The direct synthesis of alpha-aminomethylphosphonic acids. Mannich-type reactions with orthophosphorous acid", JOURNAL OF ORGANIC CHEMISTRY, vol. 31, no. 5, May 1966 (1966-05), pages 1603-1607, XP001094340, American Chemical Society, Washington, DC, US ISSN: 0022-3263, DOI: 10.1021/jo01343a067 cited in the application

## Description

### TECHNICAL FIELD

The invention relates to the suppression/prevention of nitrosamine formation in tertiary alkylamine oxide compositions by means of special polyamino methylene phosphonates and related salts. The invention also relates to tertiary alkylamine oxide compositions with reduced contents of nitrosamine. More particularly, the invention relates to the field of surfactants which are sensitive to nitrosamine contamination. These polyamino methylene phosphonates and related salts have also shown to be effective for suppression of nitrite formation in the synthesis of tertiary alkyl amine oxides.

### BACKGROUND

It is known that nitrosamines, which are generally considered to be harmful, and nitrites, which can be precursors for nitrosamines, are present in amine oxide compositions. Typically, these contaminants are present in commercial amine oxide compositions at contents between 800 and 4000 ppb. EP 0 608 873 discloses a method for the preparation of amine oxides by a reaction of an amine with hydrogen peroxide in the presence of sodium bicarbonate and various nitrosamine/nitrite inhibitors, such as sodium diethylenetriamine pentakis-(methylenephosphonate) and sodium tetraethylenepentamine heptakis-(methylenephosphonate).

Because of the carcinogenicity of nitrosamines, pressure increasingly grows to reduce their contents. Improved methods of analysis allow to determine contents of ATNC (Apparent Total Nitrosatable Content) expressed as NNO lower than 50 ppb "total NNO" and wherein nitrite, expressed as "total NO", is the sum of the nitrosamines and volatile nitrites. Consequently, the search is also started for methods to prevent the formation of these contaminants, or to suppress them as far as possible.

The present invention aims to find a solution for at least some of the abovementioned problems.

The invention aims to improve the final specifications of tertiary alkyl amine oxide products and to reduce the content of by-products. Process improvements should be economically feasible, i.e. in terms of cost and reaction time.

### SUMMARY

The invention provides a solution thereto by providing an improved method of synthesis for amine oxide compositions. Also, the invention relates to compositions with reduced contents of nitrosamine and use of an inhibitor to reduce nitrosamine contents. The inhibitor can be used for the suppression of nitrite content.

In a first aspect, the invention provides a method for the production of tertiary alkyl amine oxides with prevented and/or reduced nitrosamine formation and/or reduced nitrite formation, comprising the steps of:
during or after the production of the aforementioned tertiary alkyl amine oxide, adding a mixture comprising
(a) a carbonate or bicarbonate and
(b) a polyamino methylene phosphonate of formula (I) 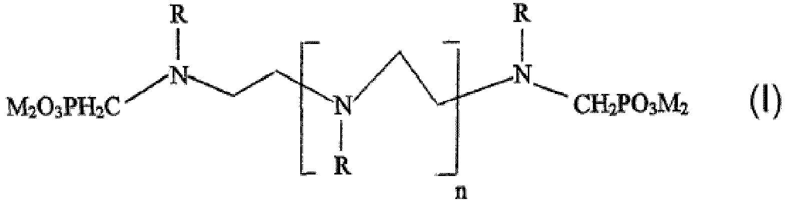
   wherein n=2-1500,
   M is hydrogen or a cation selected from alkali metals, alkaline earth metals and ammonium,
   and the R groups are equal or different from one other with at least one R group different from CH₂PO₃M₂ and selected from the following classes:
      1) CH₂PO₃M₂
      2) CH₂R' R'=CH₂OH; CHOHCH₃; CHOHCH₂Cl or CHOHCH₂OH;
      3) (CH2)ₘSO₃M wherein m = 3 or 4;
      4) CH₂CH₂R" wherein R"=CONH₂, CHO, COOR1, COOX or CN;
         where R1=CH₃ or C₂H₅;
         wherein X is hydrogen or an alkali metal or ammonium cation.

The inventors have found that with this mixture, the final specifications of tertiary alkyl amine oxides could be improved. By the improvement of the final specifications is particularly meant, the content of nitrosamines of an amine oxide composition and/or its colour stability. Preferably, also the nitrite content is reduced as a result of the use of formula (I) compound.

In a second aspect, the invention provides a composition obtained according to a method of the invention.

Compositions with reduced nitrosamine contents were obtained by the presence of the mixture. An advantage of the compositions is their stability over longer periods of time. Stability refers to the colour stability and/or stability of the content of nitrosamines. Additionally, the content of nitrite may also be reduced.

In a third aspect, the invention provides a composition comprising a tertiary alkyl amine oxide, a carbonate or bicarbonate and a polyamino methylene phosphonate of formula (I) as described above.

The tertiary alkylamine oxide compositions, in particular cocamidopropylamine oxide compositions, meet stricter final specifications in regard to nitrosamines and amido-amine residual content. Preferably, this is also the case for the nitrite content.

In a fourth aspect, the invention provides the use of the aforementioned polymethylene phosphonates for preventing or suppressing nitrosoamine formation in a tertiary alkyl amine oxide solution. Aforementioned polymethylene phosphonates can also be used for reducing the content of nitrite in a tertiary alkylamine oxide solution.

The use of the aforementioned polymethylene phosphonates has proven to be very advantageous to stabilize tertiary alkylamine oxide compositions. Strict final specifications in terms of nitrosamines and amido-amine residual content are attainable without sacrificing reaction time. Using polymethylene phosphonates as aforementioned, in the synthesis of tertiary alkyl amine oxide compositions offers as an advantage that nitrite formation is reduced.

Further improvements have been set out in the dependent claims.

### DETAILED DESCRIPTION OF THE INVENTION

Unless defined otherwise, all terms used in the description of the invention, including technical and scientific terms, have the meaning as commonly understood by the skilled person in the technical field of the invention. For a better assessment of the description of the invention, the following terms are explained explicitly.

"A", "an" and "the" refer in this document to both the singular and the plural, unless the context clearly implies otherwise. For example, "a segment" means one or more than one segment.

Where "about" or "about" are used in this document with a measurable quantity, a parameter, a time period or moment in time, and the like, then variations are meant of +/-20% or less, preferably +/-10% or less, more preferably +/-5% or less, even more preferably +/-1% or less, to the extent that such variations apply in the described invention. It should, however, be understood that the value of the quantity in which the term "about" or "around" is used, is itself specifically disclosed.

The terms "comprise", "comprising", "consist of", "consisting of", "provided with", "contain", "containing", "include", "including", "encompass", "encompassing" are synonyms and are inclusive or open terms which indicate the presence of what follows, and which do not exclude or prevent the presence of other components, features, elements, members, steps, known from and described in the prior art.

The quoting of numerical intervals by endpoints includes all integers, fractions and/or real numbers between the endpoints, these endpoints included.

Surprisingly, it has been found that specific polyamino methylene phosphonate derivatives were able to reduce nitrosamine formation and/or nitrite formation much more than what was previously possible with other inhibitors. Therefore, these polyphosphonates that can be applied for nitrosamine reduction, are the main subject of the present invention.

In general, such compounds can be the derivatives of polyamino methylene phosphonates of the following general formula: 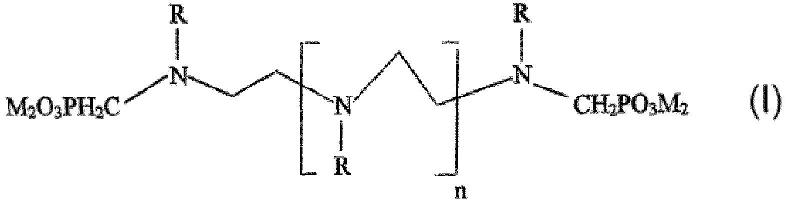
wherein n is an integer comprised between 2 and 1500, preferably between 2 and 50, more preferably 2, the endpoints included; M₂ may be hydrogen or a suitable cation such as an alkali metal, alkaline earth metal or ammonium, and each R group may be the same or different and is preferably selected from the following classes:
   1. CH₂PO₃M₂ wherein M is hydrogen or a suitable cation such as alkali metal or ammonium;
   2. CH₂R' wherein R'=CH₂OH; CHOHCH₃; CHOHCH₂Cl or CHOHCH₂OH;
   3. (CH2)ₘSO₃M wherein n = 3 or 4 where M is hydrogen or a suitable cation such as alkali metal or ammonium;
   4. CH₂CH₂R" wherein R"=CONH₂, CHO, COOR1, COOX or CN;
      where R1=CH₃ or C₂H₅;
      wherein X is hydrogen or a suitable cation such as alkali metal or ammonium;
yet at least one of the substituents R should be different from the methylene phosphonated group, i.e. other than CH₂PO₃M₂.

These polyamino methylene phosphonate compounds are described in WO 2005/073130, US 7659315 and EP 1 525 206.

Methods of preparation for these phosphonates are known. They can be prepared by means of a phosphonomethylation reaction of a polyamine or polyamine mixture, by means of a Mannich reaction as follows:

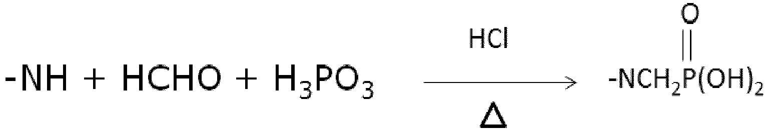

The phosphonomethylation reaction of amines according to Mannich is described in the literature, as, for example, by Moedritzer and Irani in J. Organic Chem. 31 (5), p1603-1607. A typical reaction provides that the amine is added slowly to a mixture of phosphorous acid and hydrochloric acid. The reaction mixture thus obtained is heated under reflux with addition of formaldehyde. The reaction time usually ranges between 1 and 5 hours.

Suitable polyamino methylene phosphonate derivatives of formula (I) for use in the present invention are Hydrodis WP20, WP40 and Hydrodis Hydrodis WP56, available at Giovanni Bozzetto S.p.A, Italy. Hydrodis WP20 exhibits an inhibitory effect on the formation of nitrosamines. Additional tests have shown that Hydrodis WP40 and Hydrodis WP56 exhibit an even better inhibitory effect. Further research has shown that these compounds also have a favourable effect since nitrite formation is suppressed.

By the term "inhibitory effect", "inhibition" as used herein, is meant that the additive has an impact on the formation of nitrosamines, wherein the formation of nitrosamines is prevented and/or suppressed.

The inhibitory effect was unexpected since a good effect was rather expected to occur with monomers, rather than with oligomers or polymers as is the case in the present phosphonates. A further advantage is the compatibility with the catalyst carbonate or bicarbonate. No negative impact on the colour of the end product was found.

The present invention relates to a process for the production of tertiary alkyl amine oxides with prevented and/or reduced nitrosamine formation and/or nitrite formation, comprising the steps of: during or after the production of the afore-mentioned tertiary alkyl amine oxide, adding a polyamino methylene phosphonate of formula (I) 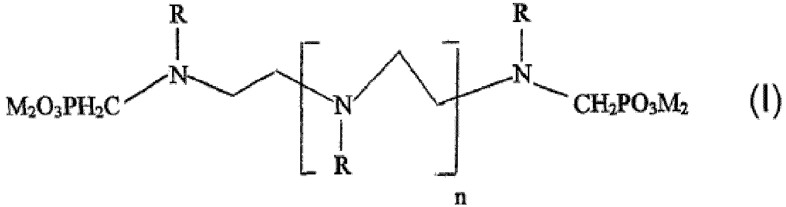
wherein n=2-1500,
M is hydrogen or a cation selected from alkali metals, alkaline earth metals and ammonium,
and the R groups are the same or different from one another with at least one R group different from CH₂PO₃M₂ and selected from the following classes:
   1) CH₂PO₃M₂
   2) CH₂R' R'=CH₂OH; CHOHCH₃; CHOHCH₂Cl or CHOHCH₂OH;
   3) (CH2)ₘSO₃M wherein m = 3 or 4;
   4) CH₂CH₂R" wherein R"=CONH₂, CHO, COOR1, COOX or CN;
      where R1=CH₃ or C₂H₅;
      wherein X is hydrogen or an alkali metal or ammonium cation.

In a preferred embodiment, n=2-50. In a preferred embodiment, n is equal to 2 and at least two R-groups differ from CH₂PO₃M₂.

In a preferred embodiment, the amine oxide is produced by reaction of a tertiary alkyl amine with hydrogen peroxide in the presence of a mixture comprising 0,05 to 20 percent by weight, based on the weight of the amine, of (a) a carbonate or bicarbonate, and (b) 0,05 to 5 percent by weight, based on the weight of the amine, of afore-mentioned polyamino methylene phosphonate.

More preferably, the mixture comprises 0,075-2 percent by weight, most preferably 0.10-0.30 percent by weight of carbonate or bicarbonate (a).

More preferably, the mixture comprises 0,5-1 percent by weight, most preferably 0.1-0.3 percent by weight of afore-mentioned polyamino methylene phosphonate (b).

Most preferably, 0,1-0,3% of carbonate or bicarbonate (a) and 0,1-0,3% of afore-mentioned polyamino methylene phosphonate (b) is used.

Preferably, 0,1% inhibitor is used, expressed with respect to the amido-amine or amine raw material for the preparation of the tertiary alkylamine oxide.

Carbonate or bicarbonate is a preferred catalyst for the conversion of a tertiary alkylamine with the aid of hydrogen peroxide to a tertiary alkylamine oxide. These catalysts are compatible with the use of afore-mentioned polyamino methylene phosphonate inhibitor.

In a preferred embodiment of a method according to the invention, the tertiary alkylamine oxide is selected from the list cocamine oxide (chemical name: cocodimethylamine oxide), cocamidopropylamine oxide (chemical name: cocoamidopropyldimethylamine oxide), laurylamidopropylamine oxide (chemical name: laurylamidopropyldimethylamine oxide), lauramine oxide (chemical name: lauryldimethylamine oxide)., myristamine oxide (myristyldimethylamine oxide), lauryl/myristylamidopropylamine oxide (lauryl/myristylamidopropyldimethylamine oxide, decylamine oxide (decyldimethylamine oxide) Mixtures of oxides from the list are also possible.

In a preferred embodiment of a method according to the invention, the tertiary alkyl amine oxide is cocamidopropylamine oxide (chemical name: cocoamidopropyldimethylamine oxide).

The structural formula for cocamidopropylamine is as follows, as shown by means of formula (II), wherein R represents a C8 to C18 alkyl.

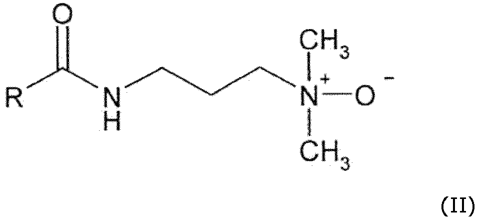

Cocamidopropylamine oxide is a non-ionic surfactant. This substance is characterized by its activity as foaming agent and foam stabilizer, as antistatic agent, as mild conditioner, and is also used for the build-up of viscosity. This substance is classified under the non-ionic surfactants and is mainly used in household dishwasher liquids, for the cleaning of hard surfaces, in chlorine bleaches, in toilet cleaners and cleaning products for cars. It is also applied in cosmetics, and mainly in body and facial cleansers and shampoos.

The synthesis of cocoamidopropylamine oxide is known. A method to produce this active substance is the reaction of cocamidopropylamine with hydrogen peroxide using sodium bicarbonate as catalyst. Hydrogen peroxide is added to a reaction mixture having a pH lower than 9 so that the peroxide would not dissolve.

In another preferred embodiment of a method according to the invention, the tertiary alkylamine is laurylamidopropylamine or lauryl/myristylamidopropylamine.

Laurylamidopropylamine has a structural formula as shown by formula (II) wherein R is a C12 alkyl residue. In lauryl/myristylamidopropylamine, the R part is a C12/C14 alkyl residue, i.e. the number of hydrocarbon atoms in the alkyl tail is 12/14.

Tertiary alkyl amines can act as precursors for the formation of nitrosamines. Once formed, they are stable and cannot be dissolved easily. The ease with which nitrosamines are formed, is dependent on the structure of the compound, the nature of the medium and the possible presence of a catalyst.

In a preferred embodiment, the tertiary alkylamine oxide is produced within a reaction time of 50 hours when a reaction temperature of 50 °C is used; and within a reaction time of 30 hours, preferably less than 25 hours, when a reaction temperature of 70 °C is used. Limiting the reaction time has an economic relevance. An increase in the reaction temperature and thus shortening the reaction time is not evident, since nitrosamine formation mostly increases at elevated temperatures.

In a preferred embodiment, at least a part of afore-mentioned polyamino methylene phosphonate derivate is added to the amine oxide before and after the production thereof (pre and post-synthesis addition).

In a preferred embodiment, the polyamino methylene phosphonate derivate was obtained by means of a two-step reaction:
step 1: reaction of N,N'-bis(2-amino-ethyl)ethane-1,2-diamine(triethylenetriamine) (I) with ethylene oxide (II) in a weight ratio (I)/(II) 1.659/1;
step 2: phosphono methylation of the product obtained in step 1 with formaldehyde and H₃PO₃ according to reaction (A)

   -NH + HCHO + H₃PO₃ -→ -NHCH₂PO(OH)₂

   wherein the weight ratio of the product obtained in step 1 / formaldehyde/phosphorous acid is 1/0,05376/1,430;
   wherein the P31-NMR analysis of the thus obtained product shows that at least 90% of the amino groups in step 2 were phosphonomethylated.

In a second aspect, the invention relates to compositions directly obtained according to a method of the invention.

In a further aspect, the invention relates to a composition comprising a tertiary alkylamine oxide, and a polyamino methylene phosphonate of formula (I) 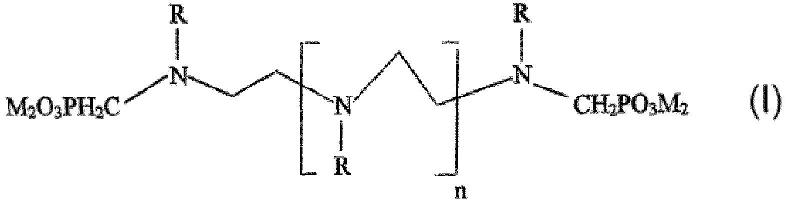
wherein n=2-1500,
M is hydrogen or a cation selected from alkali metals, alkaline earth metals and ammonium,
and the R groups are the same or different from one another with at least one R group different from CH₂PO₃M₂ and selected from the following classes:
   1) CH₂PO₃M₂
   2) CH₂R' R'=CH₂OH; CHOHCH₃; CHOHCH₂Cl or CHOHCH₂OH;
   3) (CH2)ₘSO₃M wherein m = 3 or 4;
   4) CH₂CH₂R" wherein R"=CONH₂, CHO, COOR1, COOX or CN;
      where R1=CH₃ or C₂H₅;
      wherein X is hydrogen or an alkali metal or ammonium cation.

Preferably, in a composition according to the invention, n=2-50. When n=2, preferably at least two R groups differ from CH₂PO₃M₂.

In a preferred embodiment of a composition according to the invention, the nitrosamine content is under 500 ppb and preferably lower than 100 ppb. For the measurement, gas chromatography in combination with a thermal energy analyzer (GC-TEA) is used. In measurements using this technique, the so-called apparent total N-nitroso compounds (ATNC) are determined by a chemical denitrosation and detection of the released dinitrogen(mono)oxide and reported in terms of dinitrogen(mono)oxide (nitrous oxide, NNO) in µg/kg (ppb). This content is also determined by denitrosation of an N-nitrosodiisopropanolamine (NDiPLA) calibration standard.

In a preferred embodiment of a composition according to the invention, the nitrite content is less than 2000 ppb, measured by ion chromatography (IC). Compositions obtained without the use of an inhibitor according to the invention have a nitrite content higher than 2000 ppb.

In a preferred embodiment of a composition according to the invention, in a composition with a solids content comprised between 36,0 and 38,0 Brix% measured with the aid of a refractometer, the free amido-amine content is maximum 1%, expressed in weight% with respect to the total composition, measured with a high-pressure liquid chromatograph with UV detection (UV-HPLC).

Preferably, the tertiary amine oxide is cocamidopropylamine oxide.

Cocamidopropylamine oxide is a substance which is already being used in detergent applications, but by reducing the toxicity profile, it will allow this substance to also be used in cosmetic applications, in which the contact with the human body is the most relevant.

In another preferred embodiment, the tertiary amine oxide is laurylamidopropylamine or lauryl/myristylamidopropylamine.

In a final aspect, the invention provides the use of the aforementioned polyamino methylene phosphonates, for preventing or suppressing nitrosoamine and the formation in a tertiary amine oxide solution. Afore-mentioned polyamino methylene phosphonates act as inhibitors of nitrosamine formation.

In an additional final aspect, the invention provides the use of the afore-mentioned polyamino methylene phosphonates, for suppressing the formation of nitrite in a tertiary amine oxide solution. Afore-mentioned polyamino methylene phosphonates act as inhibitors of nitrite formation.

In what follows, the invention is described with reference to non-limiting examples which illustrate the invention, and which should not be intended or interpreted as to limit the scope of the invention.

### EXAMPLES

### General method for cocamidopropylamine oxide

To a three-neck flask of 1000 ml, demi-water and citric acid were added. The acid was added in order to keep the pH of the reaction mixture below 9. Subsequently, cocamidopropylamine and hydrogen peroxide were added. The reaction mixture was heated to a desired temperature of either 50 °C or 70 °C. Upon reaching the desired reaction temperature, sodium bicarbonate was added. The pH of the reaction mixture is maintained between 7,0 and 7,5. If necessary, NaOH is added. The final product has a dry matter content of between 36,0 and 38,0 Brix%.

In the examples described below, different additives were tested for their ability to inhibit the formation of nitrosamines. Each time, during the reaction, an amount of inhibitor was added which corresponds to 0,1% by weight of the amount of added amido-amine raw material. The resulting compositions were listed in Table 1.

The nitrosamine content was determined with the aid of a chemical denitrosation technique and measurement with a thermal energy analyzer. The detection limit of the method used was 50 µg total content of N-nitroso compounds/kg.

The amido-amine residual content was determined in the final product obtained. The amine oxide was converted to amide by reflux with acetic acid anhydride whereby the amido-amine does not react. A sample is then diluted with methanol and injected onto an Ultimate 3000 high pressure liquid chromatograph. The separation took place on a reversed-phase Luna 3 U CN 100 A and the detection was done with a UV detector. The amido-amine concentration was determined using a calibration curve and standards.

### Example 1 (samples 1 and 2): polyamine methylene phosphonate

To the reaction mixture, the polyamino methylene phosphonate Hydrodis WP20 was added, in an amount of 0,1 percent by weight on amido-amine. This resulted in a low content of nitrosamines. All this could be achieved within a relatively short reaction time.

### Example 2 (samples 3 and 4): no additive

In a comparative test, no additive was added. This resulted in a higher content of nitrosamines and free amido-amine residual content.

### Example 3 (samples 5 and 6): DTPA Na

In a comparative test, 0,1 percent by weight, with respect to the amido amine, of diethylene triamine pentaacetic acid (DTPA Na) was added. By using this additive, it was, at 50 °C reaction temperature, not possible to obtain a final specification below 0,3% of amido-amine residual content within an acceptable reaction time. Increasing the reaction temperature to 70 °C made this possible, yet the colour of the final product became unacceptably high, i.e. above 100 Apha.

### Example 4 (samples 7 and 8): DTPMP

In a comparative test, diethylenetriamine penta(methylene phosphonic acid) (DTPMP) was added. The specifications related to colour and the residual content of amido-amine were achieved. At a reaction temperature of 50 °C, a long reaction time was required for this. Both samples showed an excessive content of nitrosamines. The additive is insufficiently able to inhibit the formation of nitrosamines.

### Example 5 (samples 9 and 10): EDTA

In a comparative test, ethylenediaminetetraacetic acid (EDTA) was added. The specifications related to colour and the residual content of amido-amine were achieved. At a reaction temperature of 50 °C, a long reaction time was required for this. Both samples showed an increased content of nitrosamines compared to the Hydrodis 20WP additive. The additive is insufficiently able to inhibit the formation of nitrosamines.

### Colour stability (Examples 1 and 2)

In addition to a positive influence on nitrosamines, it was also found that a method according to an embodiment of the invention results in compositions which exhibit a less pronounced increase in colour during storage at 30 °C. The products are less yellow with respect to compositions without the use of an inhibitor or even with respect to compositions with another inhibitor. This improved colour stability is an important advantage. The compositions are processed in compositions in which colour is an important aspect.

### Example 6: long term stability at room temperature

After a storage time of 10 months, measurements were again carried out on a sample produced according to the description in example 1. The results are shown in Table 2. Only a slight increase was observed in colour and nitrosamine content. This indicates an improved storage stability of a tertiary amine oxide stabilized with polyamino methylene phosphonate.

**Table 2: Long-term storage stability**

| | Start | After 10 months |
|---|---|---|
| appearance | ok | ok |
| colour | 18 | 27 |
| pH | 7,9 | 7,35 |
| dry matter | 37,7 | 37,9 |
| | | |
| % amido-amine | 0,21 | 0,14 |
| % H2O2 | 0,15 | 0,045 |
| ppb nitrosamines | 306 | 389 |

### Example 7: Production Test

As a comparative test, a production batch of cocamidopropylamine oxide was produced with and without inhibitor. As catalyst, sodium bicarbonate was used. As inhibitor, Hydrodis WP20 was used. The production batches were kept separately in the factory. Samples were taken on which the nitrosamine content was determined by GC-TEA. The results are summarized in Table 3. These show that because of the use of the inhibitor, the nitrosamine content in the final product is significantly lower, in comparison with the previously used method; more particularly, lower than 300 ppb with respect to nearly 800 ppb.

### Example 8: Production test at various temperatures

In this test, cocamidopropylamine oxide was produced at various temperatures. Other parameters such as duration of reaction, catalyst, inhibitor were kept constant. The reaction temperature was varied. The results show the temperature dependence of the nitrosamine formation. The reaction temperature is preferably kept below 70 °C, more preferably below 60 °C.

**Table 4: Production test at various temperatures**

| Sample | Temperature (°C) | Content of nitrosamines (µg/kg) |
|---|---|---|
| Euroxide CPO - 1 | 67 | 507 |
| Euroxide CPO - 2 | 56,4 | 316 |
| Euroxide CPO - 3 | 80,8 | 708 |
| Euroxide CPO - 4 | 83,2 | 846 |
| Euroxide CPO - 5 | 85 | 754 |

**Table 1: Summary of the synthesis tests with candidate inhibitors and the measurement results on the compositions**

| Parameter/Sample | 1 | 2 | 3 | 4 | 5 | 6 | 7 | 8 | 9 | 10 | 11 | 12 |
|---|---|---|---|---|---|---|---|---|---|---|---|---|
| Temp (°C) | 50 | 70 | 50 | 70 | 50 | 70 | 50 | 70 | 50 | 70 | 50 | 50 |
| Additive | Hydrodis WP20 | Hydrodis WP20 | / | / | DTPA Na | DTPA Na | DTPMP | DTPMP | EDTA | EDTA | Hydrodis WP40 | Hydrodis WP56 |
| pH | 7,7 | 7,3 | 7,9 | 7,3 | 7,3 | 7,3 | 7,5 | 7,6 | 7.7 | 7,3 | 7,3 | 7,3 |
| Content of dry matter (Brix%) | 37,7 | 37,7 | 37.2 | 37,3 | 37,8 | 38,0 | 37,8 | 37,7 | 37.4 | 36,9 | 36,9 | 36,9 |
| Content of amido-amine (%) | 0,21 | 0.27 | 0,28 | 0,34 | 0,42 | 0,16 | 0,29 | 0,14 | 0,30 | 0,23 | 0,27 | 0,29 |
| Content of nitrosamines (ppb) | 312 | 306 | 777 | 1498 | / | / | 1637 | 765 | 660 | 1550 | < 50 | <17 |
| Colour (Apha) | 18 | 27 | 24 | 39 | 27 | 147 | 50 | 24 | 27 | 37 | 16 | 18 |
| Reaction time (u) | 47 | 29 | 49 | 24 | 121 | 26 | 71 | 25 | 10 | 46 | 15 | 15 |

| | | | | | | | | | | | | |
|---|---|---|---|---|---|---|---|---|---|---|---|---|
| / = not measured | | | | | | | | | | | | |

**Table 3: Production test with inhibitor Hydrodis WP 20**

| Batch n° | Tonnage | Inhibitor (a) | Catalyst (b) | Tmax | Reaction duration (h) | Nitrosamine (µg/kg) |
|---|---|---|---|---|---|---|
| 1 (prior art) | 30 | 0% | 0,25% | 63,9 | 12 | 792 |
| 2 (invention) | 20 | 0,20% | 0,62% | 68 | 11 | 257 |
| 3 (invention) | 25 | 0,20% | 0,70% | 67,4 | 12.5 | 259 |
| 4 (invention) | 25 | 0,66% | 0,66% | 69.1 | 16 | 199 |

### Example 9: Suppression of nitrosamine and nitrite formation

Lauramine oxide (chemical name: lauryldimethylamine oxide) was made without or with inhibitor Hydrodis WP20, a polyamino methylene phosphonate according to formula I, commercially available at Bozetto. 0.13% of Hydrodis WP20 was added, expressed with respect to the amount of amine used. Tmax was not higher than 50 °C. Reaction time was maximum 17h.

The nitrite content was measured using ion chromatography. The nitrosamine content was measured with the aid of a chemical denitrosation technique and measurement with a thermal energy analyzer.

**Table 5: Production batches of lauryl amine oxide without stabilizer**

| Batch | Tmax | Reaction duration (h) | Inhibitor (%) | Nitrite (ppb) |
|---|---|---|---|---|
| R1 | | | 0 | 2196 |
| R2 | | | 0 | 1155 |
| R3 | | | 0 | 1067 |
| R4 | | | 0 | 1130 |
| R5 | | | 0 | 2990 |
| R6 | | | 0 | 2890 |
| R7 | | | 0 | 1239 |
| R8 | | | 0 | 1286 |
| R9 | | | 0 | 1223 |
| R10 | | | 0 | 873 |

**Table 6: Production batches of lauryl amine oxide with Hydrodis WP20 stabilizer**

| Batch | Tmax | Reaction duration (h) | Inhibitor (%) | Nitrite (ppb) | nitrosamine (ppb NNO) |
|---|---|---|---|---|---|
| 1 | 65,5 | 12,5 | 0,13 | 568 | <17 |
| 2 | 61,4 | 13 | 0,13 | 320 | <17 |
| 3 | 64,9 | 13,5 | 0,13 | 763 | <17 |
| 4 | 64,5 | 14 | 0,13 | 738 | not measured |
| 5 | 66,2 | 17 | 0,13 | 739 | not measured |

### Example 10: suppression of nitrite formation

Lauramine oxide was made without or with inhibitor Hydrodis WP56, a polyamino methylene phosphonate according to formula I, commercially available at Bozetto. In the case of the addition of stabilizer, 0.15-0.31% of Hydrodis WP56 was used, expressed with respect to the amount of amine used. The reaction time was 10-12 hours. The maximum temperature was 66-78°C.

With respect to synthesis batches without stabilizer (Table 5), the content of nitrite is clearly lower in batches wherein stabilizer was used in the synthesis (Table 7). The nitrite content was determined by ion chromatography.

**Table 7: Production batches of lauryl amine oxide with Hydrodis WP56 stabilizer**

| Batch | Tmax | Reaction duration (h) | Inhibitor (%) | Nitrite (ppb) |
|---|---|---|---|---|
| 11 | 70,8 | 10 | 0,31 | 492 |
| 12 | 78 | 12 | 0,31 | 286 |
| 13 | 68,7 | | 0,31 | 491 |
| 14 | 66,9 | | 0,31 | 417 |
| 15 | 66,8 | | 0,31 | 206 |
| 16 | 66,7 | | 0,15 | 761 |
| 17 | 67,3 | | 0,15 | 733 |

## Claims

1. A method for the production of tertiary amine oxides with prevented and/or reduced nitrosamine formation and/or reduced nitrite formation, comprising the steps of:
during or after the production of the afore-mentioned tertiary amine oxide, adding of a polyamino methylene phosphonate of formula (I) 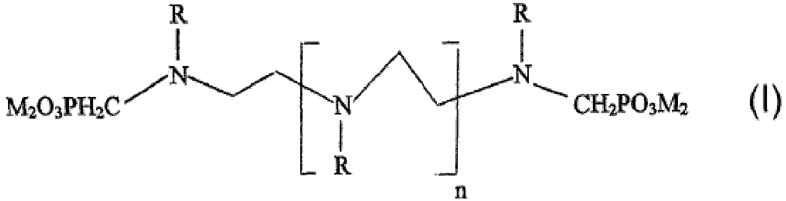
wherein n=2-1500,
M is hydrogen or a cation selected from alkali metals, alkaline earth metals and ammonium, and the R groups are the same or different from one another with at least one R group different from CH₂PO₃M₂ and selected from the following classes:
1) CH₂PO₃M₂
2) CH₂R' R'= CH₂OH; CHOHCH₃; CHOHCH₂Cl or CHOHCH₂OH;
3) (CH2)ₘSO₃M wherein m = 3 or 4;
4) CH₂CH₂R" wherein R"= CONH₂, CHO, COOR1, COOX or CN;
where R1=CH₃ or C₂H₅;
wherein X is hydrogen or an alkali metal or ammonium cation.

2. Method according to claim 1, wherein the tertiary amine oxide is selected from the list cocamine oxide, cocamidopropylamine oxide, laurylamidopropylamine oxide, lauramine oxide, myristamine oxide, lauryl/myristylamidopropylamine oxide, decylamine oxide.

3. Method according to claim 1 or 2, wherein the tertiary amine oxide is produced within a reaction time of 50 hours when a reaction temperature of 50 °C is used; and within a reaction time of 30 hours, preferably less than 25 hours, when a reaction temperature of 70 °C is used.

4. Method according to one of the preceding claims, wherein n=2-50.

5. Method according to one of the preceding claims, wherein when n=2, at least two R groups differ from CH₂PO₃M₂.

6. Method according to one of the preceding claims, wherein the amine oxide is produced by reaction of a tertiary amine with hydrogen peroxide in the presence of a mixture comprising 0,05 - 20,0% by weight, based on the weight of the amine, of (a) a carbonate or bicarbonate, and 0,05 - 5,0% by weight, based on the weight of the amine, of said polyamino methylene phosphonate (b).

7. Method according to one of the preceding claims, wherein at least a part of the polyamino methylene phosphonate (b) is added to the amine oxide before and/or after the production thereof; preferably before and after production.

8. A composition obtained according to one of the methods according to claims 1-7.

9. A composition comprising a tertiary amine oxide and a polyamino methylene phosphonate of formula (I) 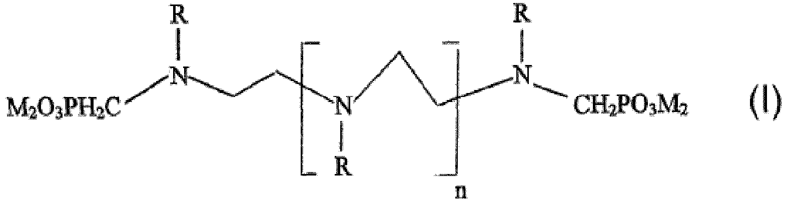
wherein n=2-1500,
M is hydrogen or a cation selected from alkali metals, alkaline earth metals and ammonium; and the R groups are the same or different from one another with at least one R group different from CH₂PO₃M₂ and selected from the following classes:
1) CH₂PO₃M₂
2) CH₂R' R'= CH₂OH; CHOHCH₃; CHOHCH₂Cl or CHOHCH₂OH;
3) (CH2)ₘSO₃M wherein m = 3 or 4;
4) CH₂CH₂R" wherein R"= CONH₂, CHO, COOR1, COOX or CN;
where R1=CH₃ or C₂H₅;
wherein X is hydrogen or an alkali metal or ammonium cation.

10. Composition according to claim 9, wherein the tertiary amine oxide is cocamidopropylamine oxide, laurylamidopropylamine or lauryl/myristylamidopropylamine.

11. Composition according to one of the preceding claims, wherein n=2-50.

12. Composition according to one of the preceding claims, wherein n=2 and at least two R groups differ from CH₂PO₃M₂.

13. Composition according to one of the preceding claims, having a nitrosamine content below 500 ppb in a solution with a tertiary amine oxide solids content comprised between 36.0-38.0 Brix%, measured by gas chromatography in combination with a thermal energy analyzer (GC-TEA).

14. Composition according to one of the preceding claims, with an amido-amine content of maximum 1%, expressed in % by weight with respect to the total composition.

15. Composition according to one of the preceding claims, with a nitrite content of maximum 2000 ppb, measured by ion chromatography (IC).

16. Use of a polyamino methylene phosphonate of formula (I) 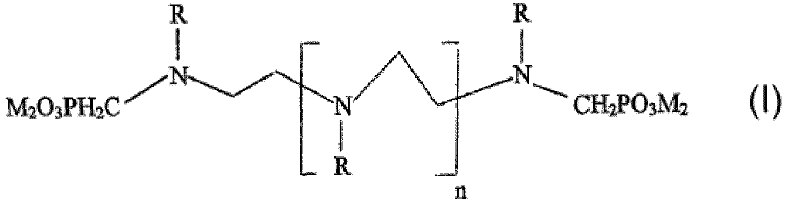
wherein n=2-1500,
M is hydrogen or a cation selected from alkali metals, alkaline earth metals and ammonium; and the R groups are the same or different from one another with at least one R group different from CH₂PO₃M₂, and selected from the following classes:
1) CH₂PO₃M₂
2) CH₂R' R'= CH₂OH; CHOHCH₃; CHOHCH₂Cl or CHOHCH₂OH;
3) (CH2)ₘSO₃M wherein m = 3 or 4;
4) CH₂CH₂R" wherein R"= CONH₂, CHO, COOR1, COOX or CN;
where R1=CH₃ or C₂H₅;
wherein X is hydrogen or an alkali metal or ammonium cation
as inhibitor for preventing or suppressing nitrosamine formation and/or reduced nitrite formation in a tertiary amine oxide solution.

## Patentansprüche

1. Verfahren zur Herstellung tertiärer Aminoxide mit unterbundener und/oder reduzierter Nitrosamin-Bildung und/oder reduzierter Nitrit-Bildung, folgende Schritte umfassend:
während oder nach der Herstellung des genannten tertiären Aminoxids Zusetzen eines Polyaminomethylenphosphonats der Formel (I) 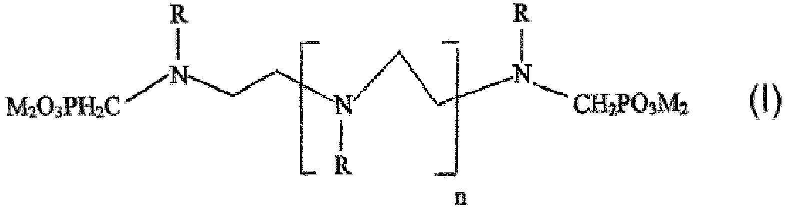
worin n=2-1500,
M Wasserstoff oder ein Kation, ausgewählt aus Alkalimetallen, Erdalkalimetallen und Ammonium, ist und die R-Gruppen gleich oder voneinander verschieden sind, wobei mindestens eine R-Gruppe von CH₂PO₃M₂ verschieden ist und aus den folgenden Klassen ausgewählt wird:
1) CH₂PO₃M₂
2) CH₂R' R'= CH₂OH; CHOHCH₃; CHOHCH₂Cl oder CHOHCH₂OH;
3) (CH2)ₘSO₃M worin m = 3 oder 4;
4) CH₂CH₂R" worin R"= CONH₂, CHO, COOR1, COOX oder CN;
wobei R1=CH₃ oder C2H5;
worin X Wasserstoff oder ein Alkalimetall- oder Ammonium-Kation ist.

2. Verfahren nach Anspruch 1, worin das tertiäre Aminoxid aus der Liste von Cocaminoxid, Cocamidopropylaminoxid, Laurylamidopropylaminoxid, Lauraminoxid, Myristaminoxid, Lauryl/Myristylamidopropylaminoxid, Decylaminoxid ausgewählt wird.

3. Verfahren nach Anspruch 1 oder 2, worin das tertiäre Aminoxid innerhalb einer Reaktionszeit von 50 Stunden erzeugt wird, wenn eine Reaktionstemperatur von 50 °C angewendet wird; und innerhalb einer Reaktionszeit von 30 Stunden, vorzugsweise weniger als 25 Stunden, wenn eine Reaktionstemperatur von 70 °C angewendet wird.

4. Verfahren nach einem der vorhergehenden Ansprüche, worin n=2-50.

5. Verfahren nach einem der vorhergehenden Ansprüche, worin, wenn n=2, mindestens zwei R-Gruppen verschieden von CH₂PO₃M₂ sind.

6. Verfahren nach einem der vorhergehenden Ansprüche, worin das Aminoxid durch Reaktion eines tertiärer Amins mit Wasserstoffperoxid in Gegenwart eines Gemischs hergestellt wird, das 0,05 - 20,0 Gewichts-%, bezogen auf das Gewicht des Amins, (a) eines Carbonats oder Bicarbonats und 0,05 - 5,0 Gewichts-%, bezogen auf das Gewicht des Amins, des Polyaminomethylenphosphonats (b) umfasst.

7. Verfahren nach einem der vorhergehenden Ansprüche, worin zumindest ein Teil des Polyaminomethylenphosphonats (b) dem Aminoxid vor und/oder nach dessen Herstellung zugesetzt wird, vorzugsweise vor und nach der Herstellung.

8. Zusammensetzung, die nach einem der Verfahren nach den Ansprüchen 1 bis 7 erhalten wird.

9. Zusammensetzung, umfassend ein tertiäres Aminoxid und ein Polyaminomethylenphosphonat der Formel (I) 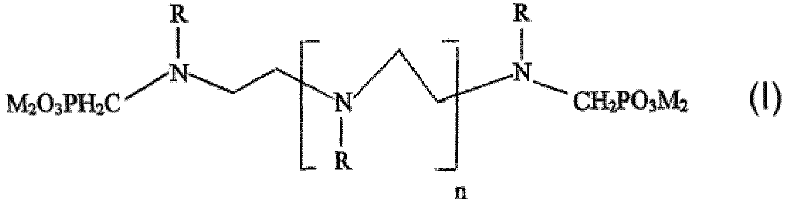
worin n=2-1500,
M Wasserstoff oder ein Kation, ausgewählt aus Alkalimetallen, Erdalkalimetallen und Ammonium, ist und die R-Gruppen gleich oder voneinander verschieden sind, worin mindestens eine R-Gruppe von CH₂PO₃M₂ verschieden und aus den folgenden Klassen ausgewählt ist:
1) CH₂PO₃M₂
2) CH₂R' R'= CH₂OH; CHOHCH₃; CHOHCH₂Cl oder CHOHCH₂OH;
3) (CH2)ₘSO₃M worin m = 3 oder 4
4) CH₂CH₂R" worin R"= CONH₂, CHO, COOR1, COOX oder CN;
wobei R1=CH₃ oder C₂H₅;
worin X Wasserstoff oder ein Alkalimetall- oder Ammonium-Kation ist.

10. Zusammensetzung nach Anspruch 9, worin das tertiäre Aminoxid Cocamidopropylaminoxid, Laurylam idopropylam in oder Lauryl/Myristylamidopropylamin ist.

11. Zusammensetzung nach einem der vorhergehenden Ansprüche, worin n=2-50.

12. Zusammensetzung nach einem der vorhergehenden Ansprüche, worin n=2 und mindestens zwei R-Gruppen verschieden von CH₂PO₃M₂ sind.

13. Zusammensetzung nach einem der vorhergehenden Ansprüche, mit einem Nitrosamin-Gehalt unter 500 ppb in einer Lösung mit einem Feststoffgehalt an tertiärem Aminoxid zwischen 36,0-38,0 %Brix, gemessen durch GasChromatographie in Kombination mit einem Thermoanalysator (GC-TEA).

14. Zusammensetzung nach einem der vorhergehenden Ansprüche, mit einem Amidoamin-Gehalt von maximal 1 %, ausgedrückt in Gewichts-% in Bezug auf die gesamte Zusammensetzung.

15. Zusam mensetzung nach einem der vorhergehenden Ansprüche, mit einem Nitrit-Gehalt von maximal 2000 ppb, gemessen durch Ionen-Chromatographie (IC).

16. Verwendung eines Polyaminomethylenphosphonats der Formel (I) 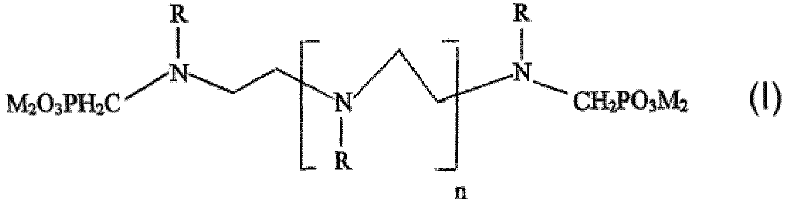
worin n=2-1500,
M Wasserstoff oder ein Kation, ausgewählt aus Alkalimetallen, Erdalkalimetallen und Ammonium, ist und die R-Gruppen gleich oder voneinander verschieden sind, worin mindestens eine R-Gruppe von CH₂PO₃M₂ verschieden und aus den folgenden Klassen ausgewählt ist:
1) CH₂PO₃M₂
2) CH₂R' R'= CH₂OH; CHOHCH₃; CHOHCH₂Cl oder CHOHCH₂OH;
3) (CH2)ₘSO₃M worin m = 3 oder 4
4) CH₂CH₂R" worin R"= CONH₂, CHO, COOR1, COOX oder CN;
wobei R1=CH₃ oder C₂H₅;
worin X Wasserstoff oder ein Alkalimetall- oder Ammonium-Kation ist,
als Inhibitor zum Unterbinden oder Unterdrücken der Nitrosamin-Bildung und/oder reduzierten Nitrit-Bildung in einer Lösung von tertiärem Aminoxid.

## Revendications

1. Procédé de production d'oxydes d'amines tertiaires avec une formation de nitrosamine empêchée et/ou réduite et/ou une formation de nitrite réduite, comprenant les étapes suivantes :
pendant ou après la production de l'oxyde d'amine tertiaire précité, l'addition d'un polyamino méthylène phosphonate de formule (I) 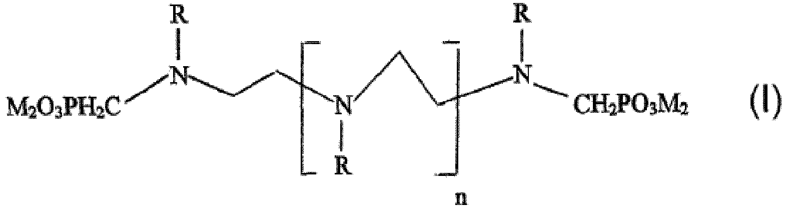
où n = 2 à 1500,
M est hydrogène ou un cation choisi parmi les métaux alcalins, les métaux alcalino-terreux et l'ammonium, et les groupes R sont identiques ou différents l'un de l'autre avec au moins un groupe R différent de CH₂PO₃M₂ et choisi dans les classes suivantes :
1) CH₂PO₃M₂
2) CH₂R' R' = CH₂OH ; CHOHCH₃ ; CHOHCH₂Cl ou CHOHCH₂OH ;
3) (CH2)ₘSO₃M où m = 3 ou 4 ;
4) CH₂CH₂R" où R" = CONH₂, CHO, COOR1, COOX ou CN ;
où R1 = CH₃ ou C₂H₅ ;
où X est hydrogène ou un métal alcalin ou un cation d'ammonium.

2. Procédé selon la revendication 1, dans lequel l'oxyde d'amine tertiaire est choisi parmi la liste constituée par l'oxyde de cocamine, l'oxyde de cocamidopropylamine, l'oxyde de laurylamidopropylamine, l'oxyde de lauramine, l'oxyde de myristamine, l'oxyde de lauryl/myristylamidopropylamine, et l'oxyde de décylamine.

3. Procédé selon la revendication 1 ou 2, dans lequel l'oxyde d'amine tertiaire est produit en un temps de réaction de moins de 50 heures lorsqu'une température de réaction de 50 °C est utilisée ; et en un temps de réaction de moins de 30 heures, de préférence inférieur à 25 heures, lorsqu'une température de réaction de 70 °C est utilisée.

4. Procédé selon l'une des revendications précédentes, dans lequel n = 2 à 50.

5. Procédé selon l'une des revendications précédentes, dans lequel lorsque n = 2, au moins deux groupes R diffèrent de CH₂PO₃M₂.

6. Procédé selon l'une des revendications précédentes, dans lequel l'oxyde d'amine est produit par réaction d'une amine tertiaire avec du peroxyde d'hydrogène en présence d'un mélange comprenant 0,05 à 20,0 % en poids, par rapport au poids de l'amine, de (a) un carbonate ou un bicarbonate, et 0,05 à 5,0 % en poids, par rapport au poids de l'amine, dudit polyamino méthylène phosphonate (b).

7. Procédé selon l'une des revendications précédentes, dans lequel au moins une partie du polyamino méthylène phosphonate (b) est ajoutée à l'oxyde d'amine avant et/ou après sa production ; de préférence avant et après la production.

8. Composition obtenue selon l'un des procédés selon les revendications 1 à 7.

9. Composition comprenant un oxyde d'amine tertiaire et un polyamino méthylène phosphonate de formule (I) 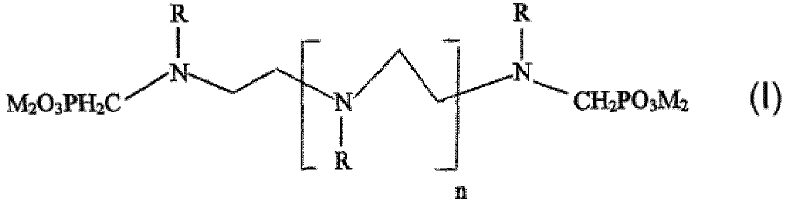
où n = 2 à 1500,
M est hydrogène ou un cation choisi parmi les métaux alcalins, les métaux alcalino-terreux et l'ammonium ; et les groupes R sont identiques ou différents l'un de l'autre avec au moins un groupe R différent de CH₂PO₃M₂ et choisi parmi les classes suivantes :
1) CH₂PO₃M₂
2) CH₂R' R' = CH₂OH ; CHOHCH₃ ; CHOHCH₂Cl ou CHOHCH₂OH ;
3) (CH2)ₘSO₃M où m = 3 ou 4
4) CH₂CH₂R" où R" = CONH₂, CHO, COOR1, COOX ou CN ;
où R1 = CH₃ ou C₂H₅ ;
où X est hydrogène ou un métal alcalin ou un cation d'ammonium.

10. Composition selon la revendication 9, dans laquelle l'oxyde d'amine tertiaire est l'oxyde de cocamidopropylamine, la laurylamidopropylamine ou la lauryl/myristylamidopropylamine.

11. Composition selon l'une des revendications précédentes, dans laquelle n = 2 à 50.

12. Composition selon l'une des revendications précédentes, dans laquelle n = 2 et au moins deux groupes R diffèrent de CH₂PO₃M₂.

13. Composition selon l'une des revendications précédentes, ayant une teneur en nitrosamine inférieure à 500 ppb dans une solution avec une teneur en matières solides d'oxyde d'amine tertiaire comprise entre 36,0 et 38,0 % Brix, mesurée par chromatographie en phase gazeuse en combinaison avec un analyseur d'énergie thermique (GC-TEA).

14. Composition selon l'une des revendications précédentes, avec une teneur en amido-amine de 1 % maximum, exprimée en % en poids par rapport à la composition totale.

15. Composition selon l'une des revendications précédentes, avec une teneur en nitrites de 2000 ppb maximum, mesurée par chromatographie ionique (IC).

16. Utilisation d'un polyamino méthylène phosphonate de formule (I) 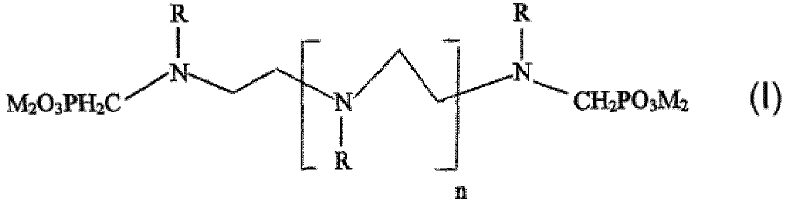
où n = 2 à 1500,
M est hydrogène ou un cation choisi parmi les métaux alcalins, les métaux alcalino-terreux et l'ammonium ; et les groupes R sont identiques ou différents l'un de l'autre avec au moins un groupe R différent de CH₂PO₃M₂, et choisi parmi les classes suivantes :
1) CH₂PO₃M₂
2) CH₂R' R' = CH₂OH ; CHOHCH₃ ; CHOHCH₂Cl ou CHOHCH₂OH ;
3) (CH2)ₘSO₃M où m = 3 ou 4
4) CH₂CH₂R" où R" = CONH₂, CHO, COOR1, COOX ou CN ;
où R1 = CH₃ ou C₂H₅ ;
où X est hydrogène ou un métal alcalin ou un cation d'ammonium
en tant qu'inhibiteur pour empêcher ou supprimer la formation de nitrosamine et/ou la formation de nitrite réduite dans une solution d'oxyde d'amine tertiaire.
